# EUROPEAN PATENT APPLICATION

(11) **EP 4 581 943 A1**
(43) Date of publication of application: **09.07.2025**
(21) Application number: 24150735.9
(22) Date of filing: 08.01.2024
(51) Int. Cl.: A24B 15/167, A24B 15/30, A24F 40/10, A24F 40/42, A61K 31/00, A61M 11/02

(54) **AEROSOLISABLE MATERIAL FOR USE IN AN ARTICLE**

(71) Applicant: BT DE Investments Inc., Wilmington, DE 19803 (US)
(72) Inventor: STROPHAIR, Oriol, London, WC2R 3LA (GB)
(74) Representative: D Young & Co LLP

(57) **Abstract**

The present invention relates to an aerosolisable material comprising at least one cannabinoid, and a solvent having a logP of 0.5 to 5.0, a viscosity of less than 400cP at ambient temperature and a boiling point of 350°C or less.

## Description

### Field of the invention

The present invention relates to an aerosolisable material, as well as an article comprising said aerosolisable material and systems using and incorporating said article.

### Background

Aerosol delivery systems which generate an aerosol for inhalation by a user are known in the art. Such systems typically comprise an aerosol generator which is capable of converting an aerosolisable material into an aerosol. In some instances, the aerosol generated is a condensation aerosol whereby an aerosolisable material is heated to form a vapor which is then allowed to condense into an aerosol. In other instances, the aerosol generated is an aerosol which results from the atomization of the aerosolisable material. Such atomization may be brought about mechanically, e.g. by subjecting the aerosolisable material to vibrations so as to form small particles of material that are entrained in airflow. Alternatively, such atomization may be brought about electrostatically, or in other ways, such as by using pressure etc.

Depending on the constituents of the aerosolisable material that are to be provided to a user, it may be preferable to formulate the aerosolisable material in a certain way. For example, it may be preferable to formulate aerosolisable materials so as to produce an aerosol with a particular profile.

### Summary

In one aspect, there is provided an aerosolisable material comprising at least one cannabinoid, and a solvent having a logP of 0.5 to 5.0, a viscosity of less than 400cP (centipoise) at ambient temperature and a boiling point of 350°C or less.

In a further aspect, there is provided an article comprising an aerosolisable material, the aerosolisable material comprising at least one cannabinoid, and a solvent having a logP of 0.5 to 5.0, a viscosity of less than 400cP (centipoise) at ambient temperature and a boiling point of 350°C or less.

These aspects and other aspects will be apparent from the following detailed description. In this regard, particular sections of the description are not to be read in isolation from other sections.

### Brief Description of the Drawings

Various embodiments will now be described in detail by way of example only with reference to the accompanying drawings in which:

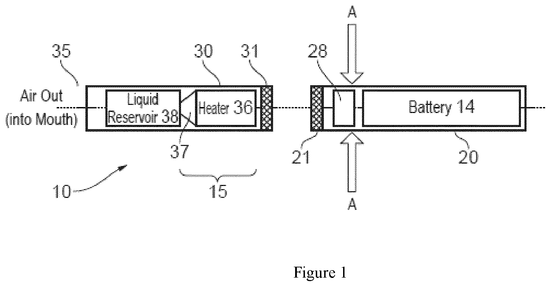

Figure 1 - Provides a schematic overview of an article, aerosol delivery device and system as described herein.

### Detailed Description

In one aspect, there is provided an aerosolisable material comprising at least one cannabinoid and a solvent,
the solvent having:
a logP of 0.5 to 5.0;
a viscosity of less than 400cP; and
a boiling point of 350°C or less..

Aerosolisable materials comprising cannabinoids can be of a relatively high viscosity. In order to reduce the viscosity of such formulations, it has been known to prepare a base formulation comprising a lower viscosity solvent, such as propylene glycol and/or a combination of different terpenes. However, providing such terpenes as solvents results in a concentration of terpenes in the formulation which is relatively high and this can overpower the user from a sensorial perspective. Moreover, the use of propylene glycol as a solvent may be less desirable to some users and so improvements are sought so as to reduce the need to use propylene glycol.

It has been found that cannabinoid formulations comprising a solvent having a logP of 0.5 to 5.0, a viscosity of less than 400cP (centipoise) at ambient temperature and a boiling point of 350°C or less, can allow for a reduced viscosity formulation to be prepared which is still capable of being aerosolized. As a result, other ingredients such as terpenes can be added at levels which are appropriate for flavour and sensation delivery, not for formulation stability/solubility. Moreover, it has been found that using a solvent having a logP of 0.5 to 5.0, a viscosity of less than 400cP and a boiling point of 350°C or less, it is possible to increase the rate of solubilisation relative to other solvents systems.

In one embodiment, the solvent has a logP of 0.5 to 5.0. In one embodiment, the solvent has a logP of 0.5 to 4.5. In one embodiment, the solvent has a logP of 0.5 to 4.0. In one embodiment, the solvent has a logP of 0.5 to 3.5. In one embodiment, the solvent has a logP of 0.5 to 3.0. In one embodiment, the solvent has a logP of 0.5 to 2.5. In one embodiment, the solvent has a logP of 0.5 to 2.0. In one embodiment, the solvent has a logP of 0.5 to 1.5. In one embodiment, the solvent has a logP of around 1.25. LogP can typically be measured using the "Shake Flask" method as described in *OECD (1995), Test No. 107: Partition Coefficient (n-octanol*/*water): Shake Flask Method, OECD Guidelines for the Testing of Chemicals, Section 1, OECD Publishing, Paris,*
In one embodiment, the solvent has a viscosity of less than 350cP at ambient temperature. In one embodiment, the solvent has a viscosity of less than 325cP at ambient temperature. In one embodiment, the solvent has a viscosity of less than 300cP at ambient temperature. In one embodiment, the solvent has a viscosity of less than 275cP at ambient temperature. In one embodiment, the solvent has a viscosity of less than 250cP at ambient temperature. In one embodiment, the solvent has a viscosity of less than 225cP at ambient temperature. In one embodiment, the solvent has a viscosity of less than 200cP at ambient temperature. In one embodiment, the solvent has a viscosity of less than 175cP at ambient temperature. In one embodiment, the solvent has a viscosity of less than 150cP at ambient temperature. In one embodiment, the solvent has a viscosity of less than 125cP at ambient temperature. In one embodiment, the solvent has a viscosity of less than 100cP at ambient temperature. In one embodiment, the solvent has a viscosity of less than 75cP at ambient temperature. In one embodiment, the solvent has a viscosity of less than 50cP at ambient temperature. In one embodiment, the solvent has a viscosity of about 40cP at ambient temperature.

Ambient temperature in the context of the present disclosure means 18 to 25°C.

Viscosity can be measured as is known to one skilled in the art using a viscometer. One example of a viscometer suitable for measuring the viscosity of an solvent as disclosed herein is a Rheolab QC rotational rheometer, available from Anton-Paar (https://www.anton-paar.com/uk-en/products/details/rotational-rheometer-rheolabqc/). In particular, suitable parameters for determining the viscosity of a solvent as disclosed herein include setting shear rate between 500 and 1000 (1/s), at a temperature of 25°C.

In one embodiment, the solvent has a boiling point of 325°C or less. In one embodiment, the solvent has a boiling point of 300°C or less.

In one embodiment, the solvent is selected from an acid ester wherein the acid ester is derived from any one of citric acid, malic acid, succinic acid, or oxalic acid. In one embodiment, the solvent is selected from an acid ester wherein the acid ester is derived from citric acid.

In one embodiment, the solvent is selected from an acid ester wherein the acid ester is derived from any one of citric acid, malic acid, succinic acid, tartaric acid or oxalic acid.

In one embodiment, the solvent is selected from an acid ester wherein the ester is a mono ester, a diester or a triester. In one embodiment, one or each ester bond may be derived from a lower alkyl group. For example, one or more ester groups may comprise a methyl group, an ethyl group, a propyl group, a butyl group, or a pentyl group.

In one embodiment, the solvent is a citrate ester with at least one ester formed with a methyl group, an ethyl group, a propyl group, a butyl group, or a pentyl group. In one embodiment, the solvent is preferably triethyl citrate.

Other suitable solvents include triacetin and propyl/isopropyl malatae diesters.

The amount of solvent, such as triethyl citrate, present in the aerosolisable material may depend on the amount of cannabinoid present, and also the presence of optional components. Typically, however, the aerosolisable material comprises solvent in an amount of from 1%w/w to 5%w/w based on the total weight of the aerosolisable material. In one embodiment, the aerosolisable material comprises solvent in an amount of from 2%w/w to 5%w/w based on the total weight of the aerosolisable material. In one embodiment, the aerosolisable material comprises solvent in an amount of from 3%w/w to 5%w/w based on the total weight of the aerosolisable material. In one embodiment, the aerosolisable material solvent in an amount of from 4%w/w to 5%w/w based on the total weight of the aerosolisable material.

In one embodiment, the aerosolisable material comprises solvent in an amount of from 1%w/w to 25%w/w based on the total weight of the aerosolisable material. In one embodiment, the aerosolisable material comprises solvent in an amount of from 1%w/w to 24%w/w based on the total weight of the aerosolisable material. In one embodiment, the aerosolisable material comprises solvent in an amount of from 1%w/w to 23%w/w based on the total weight of the aerosolisable material. In one embodiment, the aerosolisable material comprises solvent in an amount of from 1%w/w to 22%w/w based on the total weight of the aerosolisable material. In one embodiment, the aerosolisable material comprises solvent in an amount of from 1%w/w to 21%w/w based on the total weight of the aerosolisable material. In one embodiment, the aerosolisable material comprises solvent in an amount of from 1%w/w to 20%w/w based on the total weight of the aerosolisable material. In one embodiment, the aerosolisable material comprises solvent in an amount of from 1%w/w to 19%w/w based on the total weight of the aerosolisable material. In one embodiment, the aerosolisable material comprises solvent in an amount of from 1%w/w to 18%w/w based on the total weight of the aerosolisable material. In one embodiment, the aerosolisable material comprises solvent in an amount of from 1%w/w to 17%w/w based on the total weight of the aerosolisable material. In one embodiment, the aerosolisable material comprises solvent in an amount of from 1%w/w to 16%w/w based on the total weight of the aerosolisable material. In one embodiment, the aerosolisable material comprises solvent in an amount of from 1%w/w to 15%w/w based on the total weight of the aerosolisable material. In one embodiment, the aerosolisable material comprises solvent in an amount of from 1%w/w to 14%w/w based on the total weight of the aerosolisable material. In one embodiment, the aerosolisable material comprises solvent in an amount of from 1%w/w to 13%w/w based on the total weight of the aerosolisable material. In one embodiment, the aerosolisable material comprises solvent in an amount of from 1%w/w to 12%w/w based on the total weight of the aerosolisable material. In one embodiment, the aerosolisable material comprises solvent in an amount of from 1%w/w to 11%w/w based on the total weight of the aerosolisable material. In one embodiment, the aerosolisable material comprises solvent in an amount of from 1%w/w to 10%w/w based on the total weight of the aerosolisable material. In one embodiment, the aerosolisable material comprises solvent in an amount of from 1%w/w to 9%w/w based on the total weight of the aerosolisable material. In one embodiment, the aerosolisable material comprises solvent in an amount of from 1%w/w to 8%w/w based on the total weight of the aerosolisable material. In one embodiment, the aerosolisable material comprises solvent in an amount of from 1%w/w to 7%w/w based on the total weight of the aerosolisable material. In one embodiment, the aerosolisable material comprises solvent in an amount of from 1%w/w to 6%w/w based on the total weight of the aerosolisable material. In one embodiment, the aerosolisable material comprises solvent in an amount of from 1%w/w to 5%w/w based on the total weight of the aerosolisable material.

In one embodiment, the aerosolisable material comprises solvent in an amount of from 2%w/w to 25%w/w based on the total weight of the aerosolisable material. In one embodiment, the aerosolisable material comprises solvent in an amount of from 3%w/w to 25%w/w based on the total weight of the aerosolisable material. In one embodiment, the aerosolisable material comprises solvent in an amount of from 4%w/w to 25%w/w based on the total weight of the aerosolisable material. In one embodiment, the aerosolisable material comprises solvent in an amount of from 5%w/w to 25%w/w based on the total weight of the aerosolisable material. In one embodiment, the aerosolisable material comprises solvent in an amount of from 6%w/w to 25%w/w based on the total weight of the aerosolisable material. In one embodiment, the aerosolisable material comprises solvent in an amount of from 7%w/w to 25%w/w based on the total weight of the aerosolisable material. In one embodiment, the aerosolisable material comprises solvent in an amount of from 8%w/w to 25%w/w based on the total weight of the aerosolisable material. In one embodiment, the aerosolisable material comprises solvent in an amount of from 9%w/w to 25%w/w based on the total weight of the aerosolisable material. In one embodiment, the aerosolisable material comprises solvent in an amount of from 10%w/w to 25%w/w based on the total weight of the aerosolisable material. In one embodiment, the aerosolisable material comprises solvent in an amount of from 11%w/w to 25%w/w based on the total weight of the aerosolisable material. In one embodiment, the aerosolisable material comprises solvent in an amount of from 12%w/w to 25%w/w based on the total weight of the aerosolisable material. In one embodiment, the aerosolisable material comprises solvent in an amount of from 13%w/w to 25%w/w based on the total weight of the aerosolisable material. In one embodiment, the aerosolisable material comprises solvent in an amount of from 14%w/w to 25%w/w based on the total weight of the aerosolisable material. In one embodiment, the aerosolisable material comprises solvent in an amount of from 15%w/w to 25%w/w based on the total weight of the aerosolisable material. In one embodiment, the aerosolisable material comprises solvent in an amount of from 16%w/w to 25%w/w based on the total weight of the aerosolisable material. In one embodiment, the aerosolisable material comprises solvent in an amount of from 17%w/w to 25%w/w based on the total weight of the aerosolisable material. In one embodiment, the aerosolisable material comprises solvent in an amount of from 18%w/w to 25%w/w based on the total weight of the aerosolisable material. In one embodiment, the aerosolisable material comprises solvent in an amount of from 19%w/w to 25%w/w based on the total weight of the aerosolisable material. In one embodiment, the aerosolisable material comprises solvent in an amount of from 20%w/w to 25%w/w based on the total weight of the aerosolisable material.

In one embodiment, the aerosolisable material comprises solvent in an amount of about 1%w/w based on the total weight of the aerosolisable material. In one embodiment, the aerosolisable material comprises solvent in an amount of about 2%w/w based on the total weight of the aerosolisable material. In one embodiment, the aerosolisable material comprises solvent in an amount of about 3%w/w based on the total weight of the aerosolisable material. In one embodiment, the aerosolisable material solvent in an amount of about 4%w/w based on the total weight of the aerosolisable material. In one embodiment, the aerosolisable material comprises solvent in an amount of about 5%w/w based on the total weight of the aerosolisable material. In one embodiment, the aerosolisable material comprises solvent in an amount of about 6%w/w based on the total weight of the aerosolisable material. In one embodiment, the aerosolisable material comprises solvent in an amount of about 7%w/w based on the total weight of the aerosolisable material. In one embodiment, the aerosolisable material comprises solvent in an amount of about 8%w/w based on the total weight of the aerosolisable material. In one embodiment, the aerosolisable material comprises solvent in an amount of about 9%w/w based on the total weight of the aerosolisable material. In one embodiment, the aerosolisable material comprises solvent in an amount of about 10%w/w based on the total weight of the aerosolisable material. In one embodiment, the aerosolisable material comprises solvent in an amount of about 11%w/w based on the total weight of the aerosolisable material. In one embodiment, the aerosolisable material comprises solvent in an amount of about 12%w/w based on the total weight of the aerosolisable material. In one embodiment, the aerosolisable material comprises solvent in an amount of about 13%w/w based on the total weight of the aerosolisable material. In one embodiment, the aerosolisable material comprises solvent in an amount of about 14%w/w based on the total weight of the aerosolisable material. In one embodiment, the aerosolisable material comprises solvent in an amount of about 15%w/w based on the total weight of the aerosolisable material. In one embodiment, the aerosolisable material comprises solvent in an amount of about 16%w/w based on the total weight of the aerosolisable material. In one embodiment, the aerosolisable material comprises solvent in an amount of about 17%w/w based on the total weight of the aerosolisable material. In one embodiment, the aerosolisable material comprises solvent in an amount of about 18%w/w based on the total weight of the aerosolisable material. In one embodiment, the aerosolisable material comprises solvent in an amount of about 19%w/w based on the total weight of the aerosolisable material. In one embodiment, the aerosolisable material comprises solvent in an amount of about 20%w/w based on the total weight of the aerosolisable material. In one embodiment, the aerosolisable material comprises solvent in an amount of about 21%w/w based on the total weight of the aerosolisable material. In one embodiment, the aerosolisable material comprises solvent in an amount of about 22%w/w based on the total weight of the aerosolisable material. In one embodiment, the aerosolisable material comprises solvent in an amount of about 23%w/w based on the total weight of the aerosolisable material. In one embodiment, the aerosolisable material comprises solvent in an amount of about 24%w/w based on the total weight of the aerosolisable material. In one embodiment, the aerosolisable material comprises solvent in an amount of about 25%w/w based on the total weight of the aerosolisable material.

In one embodiment, the aerosolisable material has a viscosity at ambient temperature of less than 500cP. In one embodiment, the aerosolisable material has a viscosity at ambient temperature of less than 450cP. In one embodiment, the aerosolisable material has a viscosity at ambient temperature of less than 350cP. In one embodiment, the aerosolisable material has a viscosity at ambient temperature of less than 300cP. In one embodiment, the aerosolisable material has a viscosity at ambient temperature of less than 250cP. In one embodiment, the aerosolisable material has a viscosity at ambient temperature of less than 200cP. In one embodiment, the aerosolisable material has a viscosity at ambient temperature of less than 150cP. In one embodiment, the aerosolisable material has a viscosity at ambient temperature of less than 125cP.

Ambient temperature in the context of the present disclosure means 18 to 25°C.

Viscosity can be measured as is known to one skilled in the art using a viscometer. One example of a viscometer suitable for measuring the viscosity of an aerosolisable material as disclosed herein is a Rheolab QC rotational rheometer, available from Anton-Paar (https:/ /www.anton-paar.com/u k-en/prod ucts/detai Is/rotati onal-rheometer -rheolabqc/). In particular, suitable parameters for determining the viscosity of an aerosolisable material as disclosed herein include setting shear rate between 500 and 1000 (1/s), at a temperature of 25°C.

Cannabinoids are a class of natural or synthetic chemical compounds, some of which are known to act on cannabinoid receptors (i.e., CB1 and CB2) in cells that repress neurotransmitter release in the brain. Other molecular targets of cannabinoids have been identified and are discussed in Ibeas Bih, C., Chen, T., Nunn, A. V. W. et al. Molecular Targets of Cannabidiol in Neurological Disorders. Neurotherapeutics 12, 699-730 (2015*).*

Cannabinoids are cyclic molecules exhibiting particular properties such as the ability to easily cross the blood-brain barrier.

Cannabinoids may be naturally occurring (phytocannabinoids) from plants such as cannabis, (endocannabinoids) from animals, or artificially manufactured (synthesised cannabinoids).

*Cannabis sativa* species express at least 120 different phytocannabinoids (see ElSohly, M.A.; Radwan, M.M.; Gul, W.; Chandra, S.; Galal, A. Phytochemistry of Cannabis sativa L. Prog. Chem. Org. Nat. Prod. 2017, 103, 1-36) , and these may be divided into subclasses, including cannabigerols, cannabichromenes, cannabidiols, tetrahydrocannabinols, cannabinols and cannabinodiols, and other cannabinoids, such as cannabigerol (CBG), cannabigerolic acid (CBGA), cannabichromene (CBC), cannabichromenic acid (CBCA), cannabidiol (CBD), cannabidolic acid (CBDA), tetrahydrocannabinol (THC), including its isomers Δ^{6a,10a}-tetrahydrocannabinol (Δ^{6a,10a}-THC), Δ^{6a(7)}-tetrahydrocannabinol (Δ^{6a(7)}-THC), Δ⁸-tetrahydrocannabinol (Δ⁸-THC), Δ⁹-tetrahydrocannabinol (Δ⁹-THC), Δ¹⁰-tetrahydrocannabinol (Δ¹⁰-THC), Δ^{9,11}-tetrahydrocannabinol (Δ^{9,11}-THC), tetrahydrocannabinolic acid (THCA),cannabinol (CBN), cannabinolic acid (CBNA), and cannabinodiol (CBDL), cannabicyclol (CBL), cannabivarin (CBV), tetrahydrocannabivarin (THCV), cannabidivarin (CBDV), cannabichromevarin (CBCV), cannabigerovarin (CBGV), and cannabigerol monomethyl ether (CBGM).

Naturally occurring cannabinoids (phytocannabinoids) are generally present in raw plant material in their carboxylated form. These acids can be decarboxylated by moderate to high temperature (such as from heating or processing within an extraction process), UV exposure or prolonged storage to form the more active decarboxylated form. In this regard, cannabidiol (CBD) and cannabidolic acid (CBDA) are the respective decarboxylated and carboxylated forms.

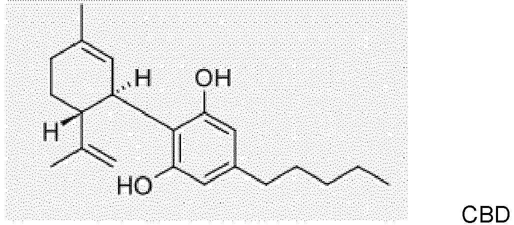

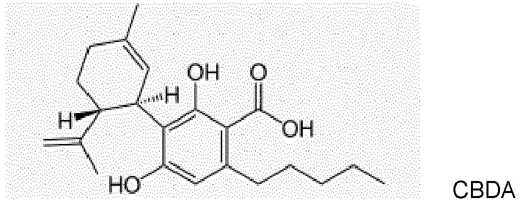

In one embodiment, the cannabinoid is selected from cannabigerol (CBG), cannabigerolic acid (CBGA), cannabichromene (CBC), cannabichromenic acid (CBCA), cannabidiol (CBD), cannabidolic acid (CBDA), tetrahydrocannabinol (THC), including its isomers Δ6a,10a-tetrahydrocannabinol (Δ6a,10a-THC), Δ6a(7)-tetrahydrocannabinol (Δ6a(7)-THC), Δ8-tetrahydrocannabinol (Δ8-THC), Δ9-tetrahydrocannabinol (Δ9-THC), Δ10-tetrahydrocannabinol (Δ10-THC), Δ9,11-tetrahydrocannabinol (Δ9,11-THC), tetrahydrocannabinolic acid (THCA),cannabinol (CBN), cannabinolic acid (CBNA), and cannabinodiol (CBDL), cannabicyclol (CBL), cannabivarin (CBV), tetrahydrocannabivarin (THCV), cannabidivarin (CBDV), cannabichromevarin (CBCV), cannabigerovarin (CBGV), and cannabigerol monomethyl ether (CBGM).

In one embodiment, the cannabinoid is selected from CBD and/or THC. In one embodiment, the cannabinoid is at least cannabidiol. In one embodiment, the cannabinoid is at least THC. In one embodiment, the cannabinoid comprises from CBD and THC.

When two cannabinoids are present in the aerosolisable material, they may be present in a 1:1 ratio on a w/w basis. In particular, the aerosolisable material may comprise CBD and THC in a 1:1 ratio on a w/w basis.

The cannabinoid may be added to the aerosolisable material as a distillate or an isolate. In particular, where the aerosolisable material comprises CBD, it may be present as CBD isolate. In particular, where the aerosolisable material comprises THC, it may be present as THC distillate.

In one embodiment, the aerosolisable material has a cannabinoid content of from about 40%w/w to about 90%w/w based on the total weight of the aerosol generating material. In one embodiment, the aerosolisable material has a cannabinoid content of from about 45%w/w to about 90%w/w based on the total weight of the aerosol generating material. In one embodiment, the aerosolisable material has a cannabinoid content of from about 50%w/w to about 90%w/w based on the total weight of the aerosol generating material. In one embodiment, the aerosolisable material has a cannabinoid content of from about 55%w/w to about 90%w/w based on the total weight of the aerosol generating material. In one embodiment, the aerosolisable material has a cannabinoid content of from about 60%w/w to about 90%w/w based on the total weight of the aerosol generating material. In one embodiment, the aerosolisable material has a cannabinoid content of from about 65%w/w to about 90%w/w based on the total weight of the aerosol generating material. In one embodiment, the aerosolisable material has a cannabinoid content of from about 70%w/w to about 90%w/w based on the total weight of the aerosol generating material. In one embodiment, the aerosolisable material has a cannabinoid content of from about 80%w/w to about 90%w/w based on the total weight of the aerosol generating material.

In one embodiment, the total amount of cannabinoid present in the aerosolisable material is from about 5 mg/ml up to about 900 mg/ml of the aerosolisable material. In one embodiment, the total amount of cannabinoid present in the aerosolisable material is from about 10 mg/ml up to about 900 mg/ml of the aerosolisable material. In one embodiment, the total amount of cannabinoid present in the aerosolisable material is from about 100 mg/ml up to about 900 mg/ml of the aerosolisable material. In one embodiment, the total amount of cannabinoid present in the aerosolisable material is from about 150 mg/ml up to about 900 mg/ml of the aerosolisable material. In one embodiment, the total amount of cannabinoid present in the aerosolisable material is from about 200 mg/ml up to about 900 mg/ml of the aerosolisable material. In one embodiment, the total amount of cannabinoid present in the aerosolisable material is from about 250 mg/ml up to about 900 mg/ml of the aerosolisable material. In one embodiment, the total amount of cannabinoid present in the aerosolisable material is from about 300 mg/ml up to about 900 mg/ml of the aerosolisable material. In one embodiment, the total amount of cannabinoid present in the aerosolisable material is from about 350 mg/ml up to about 900 mg/ml of the aerosolisable material. In one embodiment, the total amount of cannabinoid present in the aerosolisable material is from about 400 mg/ml up to about 900 mg/ml of the aerosolisable material. In one embodiment, the total amount of cannabinoid present in the aerosolisable material is from about 450 mg/ml up to about 900 mg/ml of the aerosolisable material. In one embodiment, the total amount of cannabinoid present in the aerosolisable material is from about 500 mg/ml up to about 900 mg/ml of the aerosolisable material. In one embodiment, the total amount of cannabinoid present in the aerosolisable material is from about 550 mg/ml up to about 900 mg/ml of the aerosolisable material. In one embodiment, the total amount of cannabinoid present in the aerosolisable material is from about 600 mg/ml up to about 900 mg/ml of the aerosolisable material. In one embodiment, the total amount of cannabinoid present in the aerosolisable material is from about 750 mg/ml up to about 900 mg/ml of the aerosolisable material. In one embodiment, the total amount of cannabinoid present in the aerosolisable material is from about 800 mg/ml up to about 900 mg/ml of the aerosolisable material.

In one embodiment, at least one cannabinoid is present in the aerosolisable material in an amount of about 5 mg/ml or more of the aerosolisable material. In one embodiment, at least one cannabinoid is present in the aerosolisable material in an amount of about 10 mg/ml or more of the aerosolisable material. In one embodiment, at least one cannabinoid is present in the aerosolisable material in an amount of about 15 mg/ml or more of the aerosolisable material. In one embodiment, at least one cannabinoid is present in the aerosolisable material in an amount of about 20 mg/ml or more of the aerosolisable material. In one embodiment, at least one cannabinoid is present in the aerosolisable material in an amount of about 25 mg/ml or more of the aerosolisable material. In one embodiment, at least one cannabinoid is present in the aerosolisable material in an amount of about 30 mg/ml or more of the aerosolisable material. In one embodiment, at least one cannabinoid is present in the aerosolisable material in an amount of about 35 mg/ml or more of the aerosolisable material. In one embodiment, at least one cannabinoid is present in the aerosolisable material in an amount of about 40 mg/ml or more of the aerosolisable material. In one embodiment, at least one cannabinoid is present in the aerosolisable material in an amount of about 45 mg/ml or more of the aerosolisable material. In one embodiment, at least one cannabinoid is present in the aerosolisable material in an amount of about 50 mg/ml or more of the aerosolisable material. In one embodiment, at least one cannabinoid is present in the aerosolisable material in an amount of about 55 mg/ml or more of the aerosolisable material. In one embodiment, at least one cannabinoid is present in the aerosolisable material in an amount of about 60 mg/ml or more of the aerosolisable material. In one embodiment, at least one cannabinoid is present in the aerosolisable material in an amount of about 65 mg/ml or more of the aerosolisable material. In one embodiment, at least one cannabinoid is present in the aerosolisable material in an amount of about 70 mg/ml or more of the aerosolisable material. In one embodiment, at least one cannabinoid is present in the aerosolisable material in an amount of about 75 mg/ml or more of the aerosolisable material. In one embodiment, at least one cannabinoid is present in the aerosolisable material in an amount of about 80 mg/ml or more of the aerosolisable material. In one embodiment, at least one cannabinoid is present in the aerosolisable material in an amount of about 85 mg/ml or more of the aerosolisable material. In one embodiment, at least one cannabinoid is present in the aerosolisable material in an amount of about 90 mg/ml or more of the aerosolisable material. In one embodiment, at least one cannabinoid is present in the aerosolisable material in an amount of about 95 mg/ml or more of the aerosolisable material. In one embodiment, at least one cannabinoid is present in the aerosolisable material in an amount of about 100 mg/ml or more of the aerosolisable material. In one embodiment, at least one cannabinoid is present in the aerosolisable material in an amount of about 105 mg/ml or more of the aerosolisable material. In one embodiment, at least one cannabinoid is present in the aerosolisable material in an amount of about 110 mg/ml or more of the aerosolisable material. In one embodiment, at least one cannabinoid is present in the aerosolisable material in an amount of about 115 mg/ml or more of the aerosolisable material. In one embodiment, at least one cannabinoid is present in the aerosolisable material in an amount of about 120 mg/ml or more of the aerosolisable material. In one embodiment, at least one cannabinoid is present in the aerosolisable material in an amount of about 130 mg/ml or more of the aerosolisable material. In one embodiment, at least one cannabinoid is present in the aerosolisable material in an amount of about 140 mg/ml or more of the aerosolisable material. In one embodiment, at least one cannabinoid is present in the aerosolisable material in an amount of about 150 mg/ml or more of the aerosolisable material. In one embodiment, at least one cannabinoid is present in the aerosolisable material in an amount of about 160 mg/ml or more of the aerosolisable material. In one embodiment, at least one cannabinoid is present in the aerosolisable material in an amount of about 170 mg/ml or more of the aerosolisable material. In one embodiment, at least one cannabinoid is present in the aerosolisable material in an amount of about 180 mg/ml or more of the aerosolisable material. In one embodiment, at least one cannabinoid is present in the aerosolisable material in an amount of about 190 mg/ml or more of the aerosolisable material. In one embodiment, at least one cannabinoid is present in the aerosolisable material in an amount of about 200 mg/ml or more of the aerosolisable material. In one embodiment, at least one cannabinoid is present in the aerosolisable material in an amount of about 210 mg/ml or more of the aerosolisable material. In one embodiment, at least one cannabinoid is present in the aerosolisable material in an amount of about 220 mg/ml or more of the aerosolisable material. In one embodiment, at least one cannabinoid is present in the aerosolisable material in an amount of about 230 mg/ml or more of the aerosolisable material. In one embodiment, at least one cannabinoid is present in the aerosolisable material in an amount of about 240 mg/ml or more of the aerosolisable material. In one embodiment, at least one cannabinoid is present in the aerosolisable material in an amount of about 250 mg/ml or more of the aerosolisable material. In one embodiment, at least one cannabinoid is present in the aerosolisable material in an amount of about 260 mg/ml or more of the aerosolisable material. In one embodiment, at least one cannabinoid is present in the aerosolisable material in an amount of about 270 mg/ml or more of the aerosolisable material. In one embodiment, at least one cannabinoid is present in the aerosolisable material in an amount of about 280 mg/ml or more of the aerosolisable material. In one embodiment, at least one cannabinoid is present in the aerosolisable material in an amount of about 290 mg/ml or more of the aerosolisable material. In one embodiment, at least one cannabinoid is present in the aerosolisable material in an amount of about 300 mg/ml or more of the aerosolisable material. In one embodiment, at least one cannabinoid is present in the aerosolisable material in an amount of about 310 mg/ml or more of the aerosolisable material. In one embodiment, at least one cannabinoid is present in the aerosolisable material in an amount of about 320 mg/ml or more of the aerosolisable material. In one embodiment, at least one cannabinoid is present in the aerosolisable material in an amount of about 330 mg/ml or more of the aerosolisable material. In one embodiment, at least one cannabinoid is present in the aerosolisable material in an amount of about 340 mg/ml or more of the aerosolisable material. In one embodiment, at least one cannabinoid is present in the aerosolisable material in an amount of about 350 mg/ml or more of the aerosolisable material. In one embodiment, at least one cannabinoid is present in the aerosolisable material in an amount of about 360 mg/ml or more of the aerosolisable material. In one embodiment, at least one cannabinoid is present in the aerosolisable material in an amount of about 370 mg/ml or more of the aerosolisable material. In one embodiment, at least one cannabinoid is present in the aerosolisable material in an amount of about 380 mg/ml or more of the aerosolisable material. In one embodiment, at least one cannabinoid is present in the aerosolisable material in an amount of about 390 mg/ml or more of the aerosolisable material. In one embodiment, at least one cannabinoid is present in the aerosolisable material in an amount of about 400 mg/ml or more of the aerosolisable material. In one embodiment, at least one cannabinoid is present in the aerosolisable material in an amount of about 450 mg/ml or more of the aerosolisable material. In one embodiment, at least one cannabinoid is present in the aerosolisable material in an amount of about 500 mg/ml or more of the aerosolisable material. In one embodiment, at least one cannabinoid is present in the aerosolisable material in an amount of about 550 mg/ml or more of the aerosolisable material. In one embodiment, at least one cannabinoid is present in the aerosolisable material in an amount of about 600 mg/ml or more of the aerosolisable material. In one embodiment, at least one cannabinoid is present in the aerosolisable material in an amount of about 650 mg/ml or more of the aerosolisable material. In one embodiment, at least one cannabinoid is present in the aerosolisable material in an amount of about 700 mg/ml or more of the aerosolisable material. In one embodiment, at least one cannabinoid is present in the aerosolisable material in an amount of about 750 mg/ml or more of the aerosolisable material. In one embodiment, at least one cannabinoid is present in the aerosolisable material in an amount of about 820 mg/ml or more of the aerosolisable material. In one embodiment, at least one cannabinoid is present in the aerosolisable material in an amount of about 825 mg/ml or more of the aerosolisable material. In one embodiment, at least one cannabinoid is present in the aerosolisable material in an amount of about 850 mg/ml or more of the aerosolisable material. In one embodiment, at least one cannabinoid is present in the aerosolisable material in an amount of about 875 mg/ml or more of the aerosolisable material. In one embodiment, at least one cannabinoid is present in the aerosolisable material in an amount of about 900 mg/ml of the aerosolisable material.

The aerosolisable material may further comprise at least one terpene. In one embodiment, the at least one terpene is selected from the group consisting of alpha-humulene, valencene, nerolidol, alpha-bisabolol, guaiol, caryophyllene oxide, limonene, terpinolene, beta-myrcene, beta-ocimene, camphene, alpha-pinene, beta-pinene, linalyl acetate, geranyl acetate, beta-citronellol, linalool, alpha-terpineol, eucalyptol (1,8-cineole), fenchol and camphor, (-)-isopulegol, (-)-alpha-bisabolol, (-)-beta-pinene, (-)-caryophyllene oxide, (-)-guaiol, (-)-trans-caryophyllene, (+)-cedrol, (1R)-(+)-camphor, (S)-(-)-beta-citronellol, 3-carene, alpha-cedrene, alpha-phellandrene, alpha-terpinene, borneol, caryophyllene, cedrol, cisnerolidol, citronellol, cymene, fenchone, gamma-terpinene, geraniol, Isopulegol, L-(-)- fenchone, menthol, myrcene, nerol, nerolidol 1, nerolidol 2, ocimene, ocimene 1, ocimene 2, p-cymene, pulegone, R-(+)-limonene, trans-nerolidol.

The at least one terpene may be added as part of one or more terpene "blocks". A block is a collection of terpenes which are added to an aerosolisable formulation. In some instances, the terpene block acts as a viscosity modifier. In particular, where the viscosity of the aerosolisable material without the terpene blocks added is greater than the viscosity of the terpene block, the addition of the terpene block may result in an overall reduction in the viscosity of the aerosolisable material.

In some embodiments, the terpenes in the terpene block are selected from the group consisting of alpha-humulene, valencene, nerolidol, alpha-bisabolol, guaiol, caryophyllene oxide, limonene, terpinolene, beta-myrcene, beta-ocimene, camphene, alpha-pinene, beta-pinene, linalyl acetate, geranyl acetate, beta-citronellol, linalool, alpha-terpineol, eucalyptol (1,8-cineole), fenchol and camphor, (-)- isopulegol, (-)-alpha-bisabolol, (-)-beta-pinene, (-)-caryophyllene oxide, (-)-guaiol, (-)-trans-caryophyllene, (+)-cedrol, (1R)-(+)-camphor, (S)-(-)-beta-citronellol, 3-carene, alpha-cedrene, alpha-phellandrene, alpha-terpinene, borneol, caryophyllene, cedrol, cisnerolidol, citronellol, cymene, fenchone, gamma-terpinene, geraniol, Isopulegol, L-(-)- fenchone, menthol, myrcene, nerol, nerolidol 1, nerolidol 2, ocimene, ocimene 1, ocimene 2, p-cymene, pulegone, R-(+)-limonene, trans-nerolidol.

In some embodiments, the terpenes in the terpene block are selected from the group consisting of alpha-humulene, valencene, nerolidol, alpha-bisabolol, guaiol, caryophyllene oxide, limonene, terpinolene, beta-myrcene, beta-ocimene, camphene, alpha-pinene, beta-pinene, linalyl acetate, geranyl acetate, beta-citronellol, linalool, alpha-terpineol, eucalyptol (1,8-cineole), fenchol and camphor. In some embodiments, In some embodiments, the terpenes in the terpene block are selected from the group consisting of limonene, terpinolene, beta-myrcene, beta-ocimene, camphene, alpha-pinene, beta-pinene, linalyl acetate, geranyl acetate, beta-citronellol, linalool, alpha-terpineol, eucalyptol (1,8-cineole), fenchol and camphor.

In some embodiments, the terpene block preferably comprises limonene, linalyl acetate, beta-citronellol and linalool.

In some embodiments, the terpene or the terpene block is present in the aerosolisable material in an amount of at least 0.1%w/w based on the total weight of the aerosolisable material. In one embodiment, the terpene or the terpene block is present in the aerosolisable material in an amount of at least 0.2%w/w based on the total weight of the aerosolisable material. In one embodiment, the terpene or the terpene block is present in the aerosolisable material in an amount of at least 0.3%w/w based on the total weight of the aerosolisable material. In one embodiment, the terpene or the terpene block is present in the aerosolisable material in an amount of at least 0.4%w/w based on the total weight of the aerosolisable material. In one embodiment, the terpene or the terpene block is present in the aerosolisable material in an amount of at least 0.5%w/w based on the total weight of the aerosolisable material. In one embodiment, the terpene or the terpene block is present in the aerosolisable material in an amount of at least 0.6%w/w based on the total weight of the aerosolisable material. In one embodiment, the terpene or the terpene block is present in the aerosolisable material in an amount of at least 0.7%w/w based on the total weight of the aerosolisable material. In one embodiment, the terpene or the terpene block is present in the aerosolisable material in an amount of at least 0.8%w/w based on the total weight of the aerosolisable material. In one embodiment, the terpene or the terpene block is present in the aerosolisable material in an amount of at least 0.9%w/w based on the total weight of the aerosolisable material. In one embodiment, the terpene or the terpene block is present in the aerosolisable material in an amount of at least 1.0%w/w based on the total weight of the aerosolisable material. In one embodiment, the terpene or the terpene block is present in the aerosolisable material in an amount of at least 1.1%w/w based on the total weight of the aerosolisable material.

In one embodiment, the terpene or the terpene block is present in the aerosolisable material in an amount of no greater than 5.0%w/w based on the total weight of the aerosolisable material. In one embodiment, the terpene or the terpene block is present in the aerosolisable material in an amount of at least 4.5%w/w based on the total weight of the aerosolisable material. In one embodiment, the terpene or the terpene block is present in the aerosolisable material in an amount of at least 4.0%w/w based on the total weight of the aerosolisable material. In one embodiment, the terpene or the terpene block is present in the aerosolisable material in an amount of at least 3.5%w/w based on the total weight of the aerosolisable material. In one embodiment, the terpene or the terpene block is present in the aerosolisable material in an amount of at least 3.0%w/w based on the total weight of the aerosolisable material. In one embodiment, the terpene or the terpene block is present in the aerosolisable material in an amount of at least 2.5%w/w based on the total weight of the aerosolisable material. In one embodiment, the terpene or the terpene block is present in the aerosolisable material in an amount of at least 2.0%w/w based on the total weight of the aerosolisable material. In one embodiment, the terpene or the terpene block is present in the aerosolisable material in an amount of at least 1.5%w/w based on the total weight of the aerosolisable material.

In one embodiment, the terpene or the terpene block is present in the aerosolisable material in an amount of from 0.1 to 5.0%w/w based on the total weight of the aerosolisable material. In one embodiment, the terpene or the terpene block is present in the aerosolisable material in an amount of from 0.1 to 4.5%w/w based on the total weight of the aerosolisable material. In one embodiment, the terpene or the terpene block is present in the aerosolisable material in an amount of from 0.1 to 4.0%w/w based on the total weight of the aerosolisable material. In one embodiment, the terpene or the terpene block is present in the aerosolisable material in an amount of from 0.1 to 3.5%w/w based on the total weight of the aerosolisable material. In one embodiment, the terpene or the terpene block is present in the aerosolisable material in an amount of from 0.1 to 3.0%w/w based on the total weight of the aerosolisable material. In one embodiment, the terpene or the terpene block is present in the aerosolisable material in an amount of from 0.1 to 2.5%w/w based on the total weight of the aerosolisable material. In one embodiment, the terpene or the terpene block is present in the aerosolisable material in an amount of from 0.1 to 2.0%w/w based on the total weight of the aerosolisable material. In one embodiment, the terpene or the terpene block is present in the aerosolisable material in an amount of from 0.1 to 1.5%w/w based on the total weight of the aerosolisable material.

In one embodiment, the terpene or the terpene block is present in the aerosolisable material in an amount of from 0.1 to 1.5%w/w based on the total weight of the aerosolisable material. In one embodiment, the terpene or the terpene block is present in the aerosolisable material in an amount of from 0.2 to 1.5%w/w based on the total weight of the aerosolisable material. In one embodiment, the terpene or the terpene block is present in the aerosolisable material in an amount of from 0.3 to 1.5%w/w based on the total weight of the aerosolisable material. In one embodiment, the terpene or the terpene block is present in the aerosolisable material in an amount of from 0.4 to 1.5%w/w based on the total weight of the aerosolisable material. In one embodiment, the terpene or the terpene block is present in the aerosolisable material in an amount of from 0.5 to 1.5%w/w based on the total weight of the aerosolisable material. In one embodiment, the terpene or the terpene block is present in the aerosolisable material in an amount of from 0.6 to 1.5%w/w based on the total weight of the aerosolisable material. In one embodiment, the terpene or the terpene block is present in the aerosolisable material in an amount of from 0.7 to 1.5%w/w based on the total weight of the aerosolisable material. In one embodiment, the terpene or the terpene block is present in the aerosolisable material in an amount of from 0.8 to 1.5%w/w based on the total weight of the aerosolisable material.

In one embodiment, the terpene or the terpene block is present in the aerosolisable material in an amount of from 0.9 to 1.5%w/w based on the total weight of the aerosolisable material. In one embodiment, the terpene or the terpene block is present in the aerosolisable material in an amount of from 1.0 to 1.5%w/w based on the total weight of the aerosolisable material.

In one embodiment, the total amount of the terpene or terpene block present in the aerosolisable material is up to about 60 mg/ml. In one embodiment, the total amount of the terpene or terpene block present in the aerosolisable material is up to about 50 mg/ml. In one embodiment, the total amount of the terpene or terpene block present in the aerosolisable material is up to about 40 mg/ml. In one embodiment, the total amount of the terpene or terpene block present in the aerosolisable material is up to about 30 mg/ml. In one embodiment, the total amount of the terpene or terpene block present in the aerosolisable material is up to about 20 mg/ml. In one embodiment, the total amount of the terpene or terpene block present in the aerosolisable material is up to about 15 mg/ml. In one embodiment, the total amount of the terpene or terpene block present in the aerosolisable material is up to about 14 mg/ml. In one embodiment, the total amount of the terpene or terpene block present in the aerosolisable material is up to about 13 mg/ml. In one embodiment, the total amount of the terpene or terpene block present in the aerosolisable material is up to about 12 mg/ml. In one embodiment, the total amount of the terpene or terpene block present in the aerosolisable material is up to about 11 mg/ml. In one embodiment, the total amount of the terpene or terpene block present in the aerosolisable material is up to about 10 mg/ml.

In one embodiment, the total amount of the terpene or terpene block present in the aerosolisable material is from about 1 mg/ml up to about 60 mg/ml. In one embodiment, the total amount of the terpene or terpene block present in the aerosolisable material is from about 5 mg/ml up to about 60 mg/ml. In one embodiment, the total amount of the terpene or terpene block present in the aerosolisable material is from about 10 mg/ml up to about 60 mg/ml. In one embodiment, the total amount of the terpene or terpene block present in the aerosolisable material is from about 12 mg/ml up to about 60 mg/ml. In one embodiment, the total amount of the terpene or terpene block present in the aerosolisable material is from about 15 mg/ml up to about 60 mg/ml. In one embodiment, the total amount of the terpene or terpene block present in the aerosolisable material is from about 20 mg/ml up to about 60 mg/ml. In one embodiment, the total amount of the terpene or terpene block present in the aerosolisable material is from about 30 mg/ml up to about 60 mg/ml. In one embodiment, the total amount of the terpene or terpene block present in the aerosolisable material is from about 40 mg/ml up to about 60 mg/ml. In one embodiment, the total amount of the terpene or terpene block present in the aerosolisable material is from about 50 mg/ml up to about 60 mg/ml.

In one embodiment, the total amount of the terpene or terpene block present in the aerosolisable material is from about 1 mg/ml up to about 60 mg/ml. In one embodiment, the total amount of the terpene or terpene block present in the aerosolisable material is from about 1 mg/ml up to about 50 mg/ml. In one embodiment, the total amount of the terpene or terpene block present in the aerosolisable material is from about 1 mg/ml up to about 40 mg/ml. In one embodiment, the total amount of the terpene or terpene block present in the aerosolisable material is from about 1 mg/ml up to about 30 mg/ml. In one embodiment, the total amount of the terpene or terpene block present in the aerosolisable material is from about 1 mg/ml up to about 20 mg/ml. In one embodiment, the total amount of the terpene or terpene block present in the aerosolisable material is from about 1 mg/ml up to about 15 mg/ml. In one embodiment, the total amount of the terpene or terpene block present in the aerosolisable material is from about 1 mg/ml up to about 14 mg/ml. In one embodiment, the total amount of the terpene or terpene block present in the aerosolisable material is from about 1 mg/ml up to about 13 mg/ml. In one embodiment, the total amount of the terpene or terpene block present in the aerosolisable material is from about 1 mg/ml up to about 12 mg/ml. In one embodiment, the total amount of the terpene or terpene block present in the aerosolisable material is from about 1 mg/ml up to about 11 mg/ml. In one embodiment, the total amount of the terpene or terpene block present in the aerosolisable material is from about 1 mg/ml up to about 10 mg/ml.

In one embodiment, the aerosolisable material does not comprise an additional material used as a solvent in the aerosolisable material.

In one embodiment, the aerosolisable material comprises propylene glycol in an amount of less than 25%w/w based on the total weight of the aerosolisable material. In one embodiment, the aerosolisable material comprises propylene glycol in an amount of less than 20%w/w based on the total weight of the aerosolisable material. In one embodiment, the aerosolisable material comprises propylene glycol in an amount of less than 15%w/w based on the total weight of the aerosolisable material. In one embodiment, the aerosolisable material comprises propylene glycol in an amount of less than 10%w/w based on the total weight of the aerosolisable material. In one embodiment, the aerosolisable material comprises propylene glycol in an amount of less than 5%w/w based on the total weight of the aerosolisable material. In one embodiment, the aerosolisable material comprises propylene glycol in an amount of less than 4%w/w based on the total weight of the aerosolisable material. In one embodiment, the aerosolisable material comprises propylene glycol in an amount of less than 3%w/w based on the total weight of the aerosolisable material. In one embodiment, the aerosolisable material comprises propylene glycol in an amount of less than 2%w/w based on the total weight of the aerosolisable material. In one embodiment, the aerosolisable material comprises propylene glycol in an amount of less than 1%w/w based on the total weight of the aerosolisable material. In one embodiment, the aerosolisable material comprises substantially no propylene glycol. In one embodiment, the aerosolisable material does not comprise propylene glycol.

In one embodiment, the aerosolisable material comprises glycerol in an amount of less than 25%w/w based on the total weight of the aerosolisable material. In one embodiment, the aerosolisable material comprises glycerol in an amount of less than 20%w/w based on the total weight of the aerosolisable material. In one embodiment, the aerosolisable material comprises glycerol in an amount of less than 15%w/w based on the total weight of the aerosolisable material. In one embodiment, the aerosolisable material comprises glycerol in an amount of less than 10%w/w based on the total weight of the aerosolisable material. In one embodiment, the aerosolisable material comprises glycerol in an amount of less than 5%w/w based on the total weight of the aerosolisable material. In one embodiment, the aerosolisable material comprises glycerol in an amount of less than 4%w/w based on the total weight of the aerosolisable material. In one embodiment, the aerosolisable material comprises glycerol in an amount of less than 3%w/w based on the total weight of the aerosolisable material. In one embodiment, the aerosolisable material comprises glycerol in an amount of less than 2%w/w based on the total weight of the aerosolisable material. In one embodiment, the aerosolisable material comprises glycerol in an amount of less than 1%w/w based on the total weight of the aerosolisable material. In one embodiment, the aerosolisable material comprises substantially no glycerol. In one embodiment, the aerosolisable material does not comprise glycerol.

In some embodiments, the aerosolisable material comprises water in an amount of less than 12%w/w based on the total weight of the material. In some embodiments, the aerosolisable material comprises water in an amount of less than 11%w/w based on the total weight of the material. In some embodiments, the aerosolisable material comprises water in an amount of less than 10%w/w based on the total weight of the material. In some embodiments, the aerosolisable material comprises water in an amount of less than 5%w/w based on the total weight of the material. In some embodiments, the aerosolisable material comprises water in an amount of less than 1%w/w based on the total weight of the material. In some embodiments, the aerosolisable material comprises water in an amount of less than 0.5%w/w based on the total weight of the material. In some embodiments, the aerosolisable material comprises substantially no water.

In some embodiments, the aerosolisable material does not comprise one or more of triethylene glycol, tetraethylene glycol, 1,3-butylene glycol, erythritol, meso-Erythritol, ethyl vanillate, ethyl laurate, a diethyl suberate, triethylene glycol diacetate, triacetin, a diacetin mixture, benzyl benzoate, benzyl phenyl acetate, tributyrin, lauryl acetate, lauric acid, myristic acid, and propylene carbonate.

In some embodiments, the aerosolisable material comprises one or more of triethylene glycol, tetraethylene glycol, 1,3-butylene glycol, erythritol, meso-Erythritol, ethyl vanillate, ethyl laurate, a diethyl suberate, triethylene glycol diacetate, triacetin, a diacetin mixture, benzyl benzoate, benzyl phenyl acetate, tributyrin, lauryl acetate, lauric acid, myristic acid, and propylene carbonate, in amount of less than 25%w/w based on the total weight of the aerosolisable material.

In some embodiments, the amount of one or more of of triethylene glycol, tetraethylene glycol, 1,3-butylene glycol, erythritol, meso-Erythritol, ethyl vanillate, ethyl laurate, a diethyl suberate, triethylene glycol diacetate, triacetin, a diacetin mixture, benzyl benzoate, benzyl phenyl acetate, tributyrin, lauryl acetate, lauric acid, myristic acid, and propylene carbonate, is less than 20%w/w based on the total weight of the aerosolisable material.

In some embodiments, the amount of one or more of of triethylene glycol, tetraethylene glycol, 1,3-butylene glycol, erythritol, meso-Erythritol, ethyl vanillate, ethyl laurate, a diethyl suberate, triethylene glycol diacetate, triacetin, a diacetin mixture, benzyl benzoate, benzyl phenyl acetate, tributyrin, lauryl acetate, lauric acid, myristic acid, and propylene carbonate, is less than 15%w/w based on the total weight of the aerosolisable material.

In some embodiments, the amount of one or more of of triethylene glycol, tetraethylene glycol, 1,3-butylene glycol, erythritol, meso-Erythritol, ethyl vanillate, ethyl laurate, a diethyl suberate, triethylene glycol diacetate, triacetin, a diacetin mixture, benzyl benzoate, benzyl phenyl acetate, tributyrin, lauryl acetate, lauric acid, myristic acid, and propylene carbonate, is less than 10%w/w based on the total weight of the aerosolisable material.

In some preferred embodiments, the aerosolisable material comprises one or more cannabinoids in amount of from 40%w/w to 90%w/w based on the total weight of the aerosolisable material, wherein the cannabinoids are selected from at least cannabidiol and THC, triethyl citrate in an amount of from 1%w/w to 25%w/w based on the total weight of the aerosolisable material, and one or more terpenes.

In some preferred embodiments, the aerosolisable material comprises one or more cannabinoids, wherein the cannabinoids are selected from at least cannabidiol and THC, triethyl citrate in an amount of from 1%w/w to 25%w/w based on the total weight of the aerosolisable material, one or more terpenes and substantially no propylene glycol.

In some preferred embodiments, the aerosolisable material comprises one or more cannabinoids in amount of from 40 to 90%w/w based on the total weight of the aerosolisable material, wherein the cannabinoids are selected from at least cannabidiol and THC, triethyl citrate in an amount of from 1%w/w to 25%w/w based on the total weight of the aerosolisable material, one or more terpenes and substantially no propylene glycol.

In one embodiment, the aerosolisable material is a liquid at about 25°C.

For the avoidance of doubt, combinations of the above end points are explicitly envisaged by the present disclosure.

In one embodiment, the aerosolisable material comprises one or more stabilizing components.

In one embodiment, the stabilising components are selected from antioxidants, pH modulators, chelators and radical scavengers, and combinations thereof.

In one embodiment, the one or more stabilizing components are selected from the class of compounds selected from enediols, pyrones, monoterpenoids, alpha-keto carboxylates, alpha-hydroxy carboxylic acids, esters, phenolic esters, flavonols o-glycosyls, and combinations thereof.

In one embodiment, the one or more stabilizing components are selected from the group consisting of ascorbic acid, sodium ascorbate, ethyl maltol, thymol, maltol, pyruvic acid, lactic acid, carvacrol, alpha-keto glutaric acid, alpha-keto glutarate salt, triethyl citrate, ethyl vanillate, quercetin, sucrose acetate isobutyrate, retinol, cholecalciferol, vitamin K-hydroquinone, citric acid, tartaric acid, ferulic acid, courmaric acid, propyl gallate, gallic acid, alpha lipoic acid, ascorbyl palmitate, lutein, lycopene, resveratrol, rutin, catechin, carnosol, rosmarinic acid, lipoic acid, α-resorcylic, pyrogallol, malvidin, theaflavin, apigenin, eriodictyol, glycitein, chrysoeriol, kaempferol, luteolin, vitexin, isovitexin, orientin, cannflavin A, cannflavin B, cannflavin C, delphinidin, pelargonidin, epicatechin, myricetin, chrysin, naringenin, α-terpineol, nerol, geranyl acetate, fenchol, propyl gallate, tert-butylhydroquinone, carvone and combinations thereof.

In one embodiment, the one or more stabilizing components are one or more antioxidants and one or more chelators.

In one embodiment, the one or more stabilizing components are one or more antioxidants.

The one or more antioxidants may be selected from the enediol class of compounds. For example, in one embodiment, the one or more antioxidants are selected from the group consisting of ascorbic acid, sodium ascorbate, retinol, cholecalciferol and combinations thereof.

In one embodiment, the one or more antioxidants comprise ascorbic acid and one or more additional antioxidants. In one embodiment, the one or more antioxidants comprise sodium ascorbate and one or more additional antioxidants. In one embodiment, the one or more antioxidants are ascorbic acid and/or sodium ascorbate. In one embodiment, the one or more antioxidants are ascorbic acid and sodium ascorbate. In one embodiment, the antioxidant is ascorbic acid. In one embodiment, the antioxidant is sodium ascorbate.

In one embodiment, the one or more stabilizing components are one or more chelators. In one embodiment, the one or more chelators are selected from the group consisting of ethyl maltol, and maltol.

In one embodiment, the one or more stabilizing components are each present in an amount of at least 100ppm. In one embodiment, the one or more stabilizing components are each present in an amount of at least 200ppm. In one embodiment, the one or more stabilizing components are each present in an amount of at least 300ppm. In one embodiment, the one or more stabilizing components are each present in an amount of at least 400ppm. In one embodiment, the one or more stabilizing components are each present in an amount of at least 500ppm. In one embodiment, the one or more stabilizing components are each present in an amount of at least 600ppm. In one embodiment, the one or more stabilizing components are each present in an amount of at least 700ppm. In one embodiment, the one or more stabilizing components are each present in an amount of at least 800ppm. In one embodiment, the one or more stabilizing components are each present in an amount of at least 900ppm. In one embodiment, the one or more stabilizing components are each present in an amount of at least 1000ppm. In one embodiment, the one or more stabilizing components are each present in an amount of at least 1100ppm. In one embodiment, the one or more stabilizing components are each present in an amount of at least 1200ppm. In one embodiment, the one or more stabilizing components are each present in an amount of at least 1300ppm. In one embodiment, the one or more stabilizing components are each present in an amount of at least 1400ppm. In one embodiment, the one or more stabilizing components are each present in an amount of at least 1500ppm. In one embodiment, the one or more stabilizing components are each present in an amount of at least 1600ppm. In one embodiment, the one or more stabilizing components are each present in an amount of at least 1700ppm. In one embodiment, the one or more stabilizing components are each present in an amount of at least 1800ppm. In one embodiment, the one or more stabilizing components are each present in an amount of at least 1900ppm. In one embodiment, the one or more stabilizing components are each present in an amount of at least 2000ppm. In one embodiment, the one or more stabilizing components are each present in an amount of at least 2500ppm. In one embodiment, the one or more stabilizing components are each present in an amount of at least 3000ppm. In one embodiment, the one or more stabilizing components are each present in an amount of at least 3500ppm. In one embodiment, the one or more stabilizing components are each present in an amount of at least 4000ppm. In one embodiment, the one or more stabilizing components are each present in an amount of at least 4500ppm. In one embodiment, the one or more stabilizing components are each present in an amount of at least 5000ppm. In one embodiment, the one or more stabilizing components are each present in an amount of at least 6000ppm. In one embodiment, the one or more stabilizing components are each present in an amount of at least 7000ppm. In one embodiment, the one or more stabilizing components are each present in an amount of at least 8000ppm. In one embodiment, the one or more stabilizing components are each present in an amount of at least 9000ppm. In one embodiment, the one or more stabilizing components are each present in an amount of at least 10000ppm. In one embodiment, the one or more stabilizing components are each present in an amount of at least 15000ppm.

In one embodiment, the one or more antioxidants are each present in an amount of at least 500ppm. In one embodiment, the one or more antioxidants are each present in an amount of at least 600ppm. In one embodiment, the one or more antioxidants are each present in an amount of at least 700ppm. In one embodiment, the one or more antioxidants are each present in an amount of at least 800ppm. In one embodiment, the one or more antioxidants are each present in an amount of at least 900ppm. In one embodiment, the one or more antioxidants are each present in an amount of at least 1000ppm. In one embodiment, the one or more antioxidants are each present in an amount of at least 1100ppm. In one embodiment, the one or more antioxidants are each present in an amount of at least 1200ppm. In one embodiment, the one or more antioxidants are each present in an amount of at least 1300ppm. In one embodiment, the one or more antioxidants are each present in an amount of at least 1400ppm. In one embodiment, the one or more antioxidants are each present in an amount of at least 1500ppm. In one embodiment, the one or more antioxidants are each present in an amount of at least 1600ppm. In one embodiment, the one or more antioxidants are each present in an amount of at least 1700ppm. In one embodiment, the one or more antioxidants are each present in an amount of at least 1800ppm. In one embodiment, the one or more antioxidants are each present in an amount of at least 1900ppm. In one embodiment, the one or more antioxidants are each present in an amount of at least 2000ppm.

The aerosolisable material may comprise one or more further constituents. In particular, one or more further constituents may be selected from one or more physiologically and/or olfactory active constituents, and/or one or more functional constituents.

In some embodiments, the active constituent is a physiologically active constituent and may be selected from nicotine, nicotine salts (e.g. nicotine ditartrate/nicotine bitartrate), nicotine-free tobacco substitutes, other alkaloids such as caffeine, or mixtures thereof.

In some embodiments, the active constituent is an olfactory active constituent and may be selected from a "flavour" and/or "flavourant" which, where local regulations permit, may be used to create a desired taste, aroma or sensation in a product for adult consumers. In some instances such constituents may be referred to as flavours, flavourants, cooling agents, heating agents, or sweetening agents, and may include one or more of extracts (e.g., licorice, hydrangea, Japanese white bark magnolia leaf, chamomile, fenugreek, clove, menthol, Japanese mint, aniseed, cinnamon, herb, wintergreen, cherry, berry, peach, apple, Drambuie, bourbon, scotch, whiskey, spearmint, peppermint, lavender, cardamom, celery, cascarilla, nutmeg, sandalwood, bergamot, geranium, honey essence, rose oil, vanilla, lemon oil, orange oil, cassia, caraway, cognac, jasmine, ylang-ylang, sage, fennel, piment, ginger, anise, coriander, coffee, or a mint oil from any species of the genus Mentha), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, oil, liquid, or powder.

The flavor may be added to the aerosolisable material as part of a so-called "flavour block", where one or more flavours are blended together and then added to the aerosolisable material.

The one or more other functional constituents may comprise one or more of pH regulators, colouring agents, and/or preservatives.

In particular, the pH regulator may include one or more acids selected from organic or inorganic acids. An example of an inorganic acid is phosphoric acid. The organic acid may include a carboxylic acid. The carboxylic acid may be any suitable carboxylic acid. In one embodiment the acid is a mono-carboxylic acid. In one embodiment the acid may be selected from the group consisting of ascorbic acid, acetic acid, lactic acid, formic acid, citric acid, benzoic acid, pyruvic acid, levulinic acid, succinic acid, tartaric acid, oleic acid, sorbic acid, propionic acid, phenylacetic acid, and mixtures thereof.

In some embodiments, an acceptable turbidity is achieved without the use of functional constituents which influence the stability of the aerosolisable material. For example, it may be possible to decrease the turbidity of a liquid system by introducing surface active constituents which serve to improve the emulsification/dispersion of one or more of the constituents. However, it may not be desirable to include such functional constituents due to user acceptability. Therefore, in some embodiments, the aerosolisable material does not comprise a surface active constituent. Examples of surface active constituents include medium chain triglycerides (MCT) and tocopherol acetate.

Alternatively, it can be desirable to improve the turbidity of the aerosolisable material by using a functional additive which does not negatively influence the stability or user perception. An example of such a functional additive which may be used in this regard is benzyl alcohol and functional derivatives thereof. In one embodiment, the aerosolisable material comprises benzyl alcohol and functional derivatives thereof. Functional derivatives of benzyl alcohol include compounds wherein the benzyl group is further substituted with an C₁-C₃ alkyl group.

In some embodiments, an acceptable turbidity is achieved without the use of any/significant amounts of water. In this regard, whilst water may otherwise assist in the preparation of aerosolisable materials since water containing materials may have a lower viscosity and therefore may be transferred more easily to an aerosol generating component, it has been found in the context of the present disclosure that water can negatively influence the stability of the aerosolisable material.

In one aspect, there is provided an article for use with an aerosol generating system, the article an aerosolisable material comprising at least one terpene, and triethyl citrate.

The article may be a container, such as a bottle, or may be a component for use with an aerosol provision device.

For example, the article may comprise an area (store) for receiving the aerosolisable material defined herein, an aerosol generating component, an aerosol generating area, and/or a mouthpiece.

In some embodiments, there is provided an article for use with an aerosol provision system, the article comprising a store comprising an aerosolisable material as defined herein, an aerosol generating component (such as a heater), an aerosol generating area, a transport element, and a mouthpiece.

Aerosolisable material may be transferred from the store for receiving an aerosolisable material to the aerosol generating component via a transport element, such as a wick, pump or the like. The skilled person is able to select suitable transport elements depending on the type of aerosolisable material that is to be transported and the rate at which it must be supplied. Particular mention may be made of transport elements, such as wicks, formed from fibrous materials, foamed materials, sintered materials, woven and non-woven materials.

An airflow pathway typically extends through the article (optionally via the device) to an outlet. The pathway is oriented such that generated aerosol is entrained in the airflow such that it can be delivered to the outlet for inhalation by a user.

In one embodiment, the aerosol generating component is a heater.

Typically, the area for receiving an aerosolisable material will allow for the article to be refilled with aerosolisable material as the aerosolisable material is depleted during use.

Figure 1 is a highly schematic diagram (not to scale) of an example aerosol provision system, such as an e-cigarette 10, to which embodiments are applicable. The e-cigarette has a generally cylindrical shape, extending along a longitudinal axis indicated by a dashed line (although aspects of the invention are applicable to e-cigarettes configured in other shapes and arrangements), and comprises two main components, namely an aerosol provision device 20 and an article 30.

The article 30 includes a store for aerosolisable material (source liquid) 38 containing an aerosolisable material (source liquid) from which an aerosol is to be generated. The article 30 further comprises an aerosol generating component (heating element or heater) 36 for heating aerosolisable material to generate the aerosol. A transport element or wicking element or wick 37 is provided to deliver aerosolisable material from the store 38 to the heating element 36. A part or parts of the wick 37 are in fluid communication with aerosolisable material in the store 38 and by a wicking or capillary action aerosolisable material is drawn along or through the wick 37 to a part or parts of the wick 37 which are in contact with the heater 36.

Vaporization of the aerosolisable material occurs at the interface between or proximity to the wick 37 and the heater 36 by the provision of heat energy to the aerosolisable material to cause evaporation, thus generating the aerosol. The aerosolisable material, the wick 37 and the heater 36 may be collectively referred to as an aerosol or vapour source. The wick 37 and the heater 36 may be collectively referred to as a vaporizer or an atomiser 15.

Typically a single wick will be present, but it is envisaged that more than one wick could be present, for example, two, three, four or five wicks.

As described above, the wick may be formed a ceramic material. In one embodiment, the The ceramic material may comprise sintered ceramic, sintered fibers/powders, or a combination of the two.

The (or at least one of/all of the) sintered wick(s) may have deposited thereon/embedded therein an electrically resistive heater. Such a heater may be formed from heat conducting alloys such as NiCr alloys. Alternatively, the sintered material may have such electrical properties such that when a current is passed there through, it is heated. Thus, the aerosol generating component and the wick may be considered to be integrated. In some embodiments, the aerosol generating component and the wick are formed from the same material and form a single component.

In some embodiments, the wick is formed from a sintered metal material and is generally in the form of a planar sheet. Thus, the wick element may have a substantially thin flat shape. For example it may be considered as a sheet, layer, film, substrate or the like. By this it is meant that a thickness of the wick is less or very much less than at least one of the length and the width of the wick. Thus, the wick thickness (its smallest dimension) is less or very much less than the longest dimension.

The wick may be made of a homogenous, granular, fibrous or flocculent sintered metal(s) so as to form said capillary structure. Wick elements can be made from a conductive material which is a nonwoven sintered porous web structure comprising metal fibres, such as fibres of stainless steel. For example, the stainless steel may be AISI (American Iron and Steel Institute) 316L (corresponding to European standard 1.4404). The material's weight may be in the range of 100 - 300 g/m².

Where the wick is generally planar, the thickness of the wick may be in the range of 75 - 250 µm. A typical fibre diameter may be about 12 µm, and a typical mean pore size (size of the voids between the fibres) may be about 32 µm. An example of a material of this type is Bekipor (RTM) ST porous metal fibre media manufactured by NV Bekaert SA, Belgium, being a range of porous nonwoven fibre matrix materials made by sintering stainless steel fibres.

Note also that while the material is described as planar, this refers to the relative dimensions of the sheet material and the wick (a thickness many times smaller than the length and/or width) but does not necessarily indicate flatness, in particular of the final wick made from the material. A wick may be flat but might alternatively be formed from sheet material into a non-flat shape such as curved, rippled, corrugated, ridged, formed into a tube or otherwise made concave and/or convex.

The wick element may have various properties. It is formed from a porous material to enable the required wicking or capillary effect for drawing source liquid through it from an store for aerosolisable material (where the wick meets the aerosolisable material at a store contact site) to the vaporisation interface. Porosity is typically provided by a plurality of interconnected or partially interconnected pores (holes or interstices) throughout the material, and open to the outer surface of the material. Any level of porosity may be employed depending on the material, the size of the pores and the required rate of wicking. For example, a porosity of between 30% and 85% might be selected, such as between 40% and 70%, between 50% and 80%, between 35% and 75% or between 40% and 75%. This might be an average porosity value for the whole wick element, since porosity may or may not be uniform across the wick. For example, pore size at the store contact site might be different from pore size nearer to the heater.

It is useful for the wick to have sufficient rigidity to support itself in a required within the article. For example, it may be mounted at or near one or two edges and be required to maintain its position substantially without flexing, bending or sagging.

As an example, porous sintered ceramic is a useful material to use as the wick element. Any ceramic with appropriate porosity may be used. If porous ceramic is chosen as the porous wick material, this is available as a powder which can be formed into a solid by sintering (heating to cause coalescence, possibly under applied pressure). Sintering then solidifies the ceramic to create the porous wick.

In some embodiments, the wick and heater are integrated. Moreover, in such embodiments, the wick is preferably formed of a porous ceramic having the heater deposited or otherwise arranged on the surface. The heater may be positioned on a surface of the wick which is not in contact with the aerosolisable material in the storage area. For example, where the wick takes a cuboidal shape, the heater is deposited on a wick surface which is opposite to a surface of the wick which projects towards the storage area for aerosolisable material. Moreover, the surface upon which the heater is located may positioned within the article directly opposite an air inlet into the article.

In one embodiment, the heater provides a power output of from 4W to 8W. In one embodiment, the heater provides a power output of from 4.5W to 7.5W. In one embodiment, the heater provides a power output of from 5W to 7W. In one embodiment, the heater provides a power output of from 5.5W to 7W. In one embodiment, the heater provides a power output of about 6.5W.

The article 30 further includes a mouthpiece 35 having an opening through which a user may inhale the aerosol generated by the vaporizer 15. The aerosol for inhalation may be described as an aerosol stream or inhalable airstream.

The aerosol delivery device 20 includes a power source (a re-chargeable cell or battery 14, referred to herein after as a battery) to provide power for the e-cigarette 10, and a controller (printed circuit board (PCB)) 28 and/or other electronics for generally controlling the e-cigarette 10. The aerosol delivery device can therefore also be considered as a battery section, or a control unit or section.

During operation of the device, the controller will determine that a user has initiated a request for the generation of an aerosol. This could be done via a button on the device which sends a signal to the controller that the aerosol generator should be powered. Alternatively, a sensor located in or proximal to the airflow pathway could detect airflow through the airflow pathway and convey this detection to the controller. A sensor may also be present in addition to the presence of a button, as the sensor may be used to determine certain usage characteristics, such as airflow, timing of aerosol generation etc.

For example, in use, when the heater 36 receives power from the battery 14, as controlled by the circuit board 28 possibly in response to pressure changes detected by an air pressure sensor (not shown), the heater 36 vaporizes aerosolisable material delivered by the wick 37 to generate the aerosol, and this aerosol stream is then inhaled by a user through the opening in the mouthpiece 35. The aerosol is carried from the aerosol source to the mouthpiece 35 along an air channel (not shown in Figure 1) that connects the aerosol source to the mouthpiece opening as a user inhales on the mouthpiece.

In this particular example, the device 20 and article 30 are detachable from one another by separation in a direction parallel to the longitudinal axis, as shown in Figure 1, but are joined together when the system 10 is in use by cooperating engagement elements 21, 31 (for example, a screw, magnetic or bayonet fitting) to provide mechanical and electrical connectivity between the device 20 and the article 30, in particular connecting the heater 36 to the battery 14. The battery may be charged as is known to one skilled in the art.

In some embodiments, the article comprises/forms a sealed container. For example, the sealed container may be hermetically sealed. The hermetically sealed container may comprise a blister pack with one or more hermetically sealed compartments for storage of one or more articles comprising the aerosolisable material described herein.

In some embodiments, the article comprises a housing within which the aerosolisable material is contained. The housing may be transparent such that the aerosolisable material can be viewed from outside of the housing. It may also be that the housing has a degree of opacity such that the passage of light through the housing is limited. This can be important so as to prevent light (such as ultra violet light) from entering the housing and compromising the stability of the aerosolisable material.

In a further aspect there is provided an aerosol provision system comprising an aerosol provision device and an article as defined herein.

### Examples

### Example 1 - solubility of CBG

The solubility of CBG in a range of solvent systems was tested at ambient temperature (approx. 24°C). Solvent systems were prepared and then CBG was added in a step-wise fashion and mixed until dissolved (according to visual inspection). The time taken for each addition to be solubilized was recorded.

| **PG** | | | | | | | |
|---|---|---|---|---|---|---|---|
| Intended Solvent Mass / (g) | | 40.0000 | | | | | |
| Actual Solvent Mass / (g) | | 40.0621 | | | | | |
| Mass Added / (g) | Cumulativ e Mass / (g) | Total Mass / (g) | Concentrati on / (%w/w) | Start Time / (HH:MM ) | End Time / (HH:MM ) | Total Time / (HH:MM ) | Pass / Fail |
| 1.0052 | 1.0052 | 41.0673 | 2.4477 | 13:24 | 13:55 | 00:31 | Pass |
| 1.0097 | 2.0149 | 42.0770 | 4.7886 | 13:59 | 14:29 | 00:30 | Pass |
| 1.0369 | 3.0518 | 43.1139 | 7.0785 | 14:29 | 15:09 | 00:40 | Pass |
| 1.0219 | 4.0737 | 44.1358 | 9.2299 | 15:10 | 16:06 | 00:56 | Pass |
| 1.3088 | 5.3825 | 45.4446 | 11.8441 | 16:07 | 17:15 | 01:08 | Pass |
| Although the solvent system enabled solubilisation of the CBG, the time taken to solubilize approximately 10% CBG was in excess of 2 hours. | | | | | | | |

| **PG:VG 70:30** | | | | | | | |
|---|---|---|---|---|---|---|---|
| Intended Solvent Mass / (g) | | 40.0000 | | | | | |
| Actual Solvent Mass / (g) | | 40.0303 | | | | | |
| Mass Added / (g) | Cumulati ve Mass / (g) | Total Mass / (g) | Concentra tion / (%w/w) | S5rt Time / (HH:MM ) | End Time / (HH:MM ) | Total Time / (HH:MM ) | Pass / Fail |
| 0.9986 | 0.9986 | 41.0289 | 2.4339 | 13:41 | 15:16 | 01:35 | Pass |
| 1.0566 | 2.0552 | 42.0855 | 4.8834 | 15:19 | 16:22 | 01:03 | Pass |
| 1.0314 | 3.0866 | 43.1169 | 7.1587 | 16:23 | 17:15 | 00:52 | Fail |
| The solvent system was not successful, since it could not sustain greater than approx.. 5%w/w CBG and the time taken to solubilize approximately 5% CBG was in excess of 2 hours. | | | | | | | |

| **Triethyl citrate** | | | | | | | |
|---|---|---|---|---|---|---|---|
| Intended Solvent Mass / (g) | | 40.0000 | | | | | |
| Actual Solvent Mass / (g) | | 40.0215 | | | | | |
| Mass Added / (g) | Cumulative Mass / (g) | Total Mass / (g) | Concentration / (%w/w) | Start Time / (HH:MM) | End Time / (HH:MM) | Total Time / (HH:MM) | Pass / Fail |
| 1.0560 | 1.0560 | 41.0775 | 2.5708 | 13:29 | 13:55 | 00:26 | Pass |
| 1.0360 | 2.0920 | 42.1135 | 4.9675 | 14:02 | 14:29 | 00:27 | Pass |
| 1.0446 | 3.1366 | 43.1581 | 7.2677 | 14:34 | 15:04 | 00:30 | Pass |
| 1.0507 | 4.1873 | 44.2088 | 9.4716 | 15:05 | 15:26 | 00:21 | Pass |
| The solvent system enabled solubilisation of the CBG, and the time taken to solubilize approximately 10% CBG was in less than 2 hours. | | | | | | | |

### Example 2 - solubility of CBC

A similar approach was taken as for example 1, but this time CBC was assessed.

| **PG** | | | | | | | |
|---|---|---|---|---|---|---|---|
| Intended Solvent Mass / (g) | | 30.0000 | | | | | |
| Actual Solvent Mass / (g) | | 30.0218 | | | | | |
| Mass Added / (g) | Cumulativ e Mass / (g) | Total Mass / (g) | Concentrati on / (%w/w) | Start Time / (HH:MM ) | End Time / (HH:MM ) | Total Time / (HH:MM ) | Pass / Fail |
| 0.7951 | 0.7951 | 30.8169 | 2.5801 | 09:44 | 09:59 | 00:15 | Pass |
| 1.0162 | 1.8113 | 31.8331 | 5.6900 | 10:01 | 10:21 | 00:20 | Pass |
| 0.9936 | 2.8049 | 32.8267 | 8.5446 | 10:23 | 10:52 | 00:29 | Pass |
| 0.8693 | 3.6742 | 33.6960 | 10.9040 | 10:56 | 11:11 | 00:15 | Pass |
| Although the solvent system enabled solubilisation of the CBC, the time taken to solubilize approximately 10% CBC was in excess of 1 hour. | | | | | | | |

| **PG:VG 70:30** | | | | | | | |
|---|---|---|---|---|---|---|---|
| Intended Solvent Mass / (g) | | 30.0000 | | | | | |
| Actual Solvent Mass / (g) | | 30.0416 | | | | | |
| Mass Added / (g) | Cumulati ve Mass / (g) | Total Mass / (g) | Concentra tion / (%w/w) | Start Time / (HH:MM ) | End Time / (HH:MM ) | Total Time / (HH:MM ) | Pass / Fail |
| 0.9393 | 0.9393 | 30.9809 | 3.0319 | 09:49 | 11:33 | 01:44 | Fail |
| The solvent system was not successful. | | | | | | | |

| **TEC** | | | | | | | |
|---|---|---|---|---|---|---|---|
| Intended Solvent Mass / (g) | | 30.0000 | | | | | |
| Actual Solvent Mass / (g) | | 30.0116 | | | | | |
| Mass Added / (g) | Cumulativ e Mass / (g) | Total Mass / (g) | Concentratio n / (%w/w) | Start Time / (HH:MM ) | End Time / (HH:MM ) | Total Time / (HH:MM ) | Pass / Fail |
| 0.7050 | 0.7050 | 30.7166 | 2.2952 | 09:38 | 09:52 | 00:14 | Pass |
| 1.0088 | 1.7138 | 31.7254 | 5.4020 | 09:55 | 10:02 | 00:07 | Pass |
| 1.0254 | 2.7392 | 32.7508 | 8.3638 | 10:04 | 10:10 | 00:06 | Pass |
| 0.9947 | 3.7339 | 33.7455 | 11.0649 | 10:12 | 10:30 | 00:18 | Pass |
| The solvent system enabled solubilisation of the CBC, and the time taken to solubilize approximately 10% CBC was in less than 1 hour. | | | | | | | |

Examples 1 and 2 show that a solvent system with a suitable viscosity, as well as a suitable logP value, is able to solubilize various cannabinoids more effectively and more quickly than other known solvent systems typically used in aerosol generating systems.

The various embodiments described herein are presented only to assist in understanding and teaching the claimed features. These embodiments are provided as a representative sample of embodiments only, and are not exhaustive and/or exclusive. It is to be understood that advantages, embodiments, examples, functions, features, structures, and/or other aspects described herein are not to be considered limitations on the scope of the invention as defined by the claims or limitations on equivalents to the claims, and that other embodiments may be utilised and modifications may be made without departing from the scope of the claimed invention. Various embodiments of the invention may suitably comprise, consist of, or consist essentially of, appropriate combinations of the disclosed elements, components, features, parts, steps, means, etc., other than those specifically described herein. In addition, this disclosure may include other inventions not presently claimed, but which may be claimed in future.

## Claims

1. An aerosolisable material comprising at least one cannabinoid, and a solvent having a logP of 0.5 to 5.0, a viscosity of less than 400cP (centipoise) at ambient temperature and a boiling point of 350°C or less.

2. The aerosolisable material according to claim 1, wherein the aerosolisable material comprises one or more cannabinoids selected from cannabidiol, THC and mixtures thereof.

3. The article according to claim 2, wherein the cannabinoid is at least cannabidiol.

4. The article according to claim 2, wherein the cannabinoid is at least THC.

5. The article according to claim 2, wherein the cannabinoid is at least cannabidiol and THC.

6. The aerosolisable material according to any one of the proceeding claims, wherein the aerosolisable material has a viscosity at ambient temperature of less than 400cP.

7. The aerosolisable material according to claim 6, wherein the aerosolisable material has a viscosity at ambient temperature of less than 300cP.

8. The aerosolisable material according to claim 7, wherein the aerosolisable material has a viscosity at ambient temperature of less than 200cP.

9. The aerosolisable material according to any one of the proceeding claims, wherein the aerosolisable material does not further comprises an additional carrier constituent.

10. The aerosolisable material according to claim 9, wherein the carrier constituent comprises one or more of propylene glycol and glycerol.

11. The aerosolisable material according to any one of the proceeding claims, wherein the aerosolisable material further comprises one or more terpenes.

12. The aerosolisable material according to any one claims 1 to 11, wherein the solvent is present in an amount of from about 1 to 25%w/w by total weight of the aerosolisable material.

13. The aerosolisable material according to claim 12, wherein the solvent is present in an amount of from 2 to 4 %w/w by total weight of the aerosolisable material, preferably wherein the solvent is present in an amount of about 3 %w/w by total weight of the aerosolisable material.

14. The aerosolisable material according to any one of claims 1 to 13, wherein the solvent is triethyl citrate.

15. An article comprising an aerosolisable material as defined in any one of claims 1 to 14.
